# EUROPEAN PATENT SPECIFICATION

(11) **EP 1 690 862 B1**
(45) Date of publication and mention of the grant of the patent: **08.07.2009**
(21) Application number: 06101214.2
(22) Date of filing: 02.02.2006
(51) Int. Cl.: C07D 407/04, A61K 8/49, A61Q 19/08

(54) **Fatty acid monoesters of sorbityl furfural and compositions for cosmetic and dermatological use**
Fettsäure Monoester von Furfurylidensorbitol und Zusammensetzungen zur kosmetischen oder dermatologischen Verwendung
Sorbitol-furfural mono-estérifiés par des acides gras et compositions à usage cosmétique et dermatologique

(30) Priority: 03.02.2005 IT MI20050151
(43) Date of publication of application: 16.08.2006
(73) Proprietor: Giuliani S.p.A., 20129 Milano (IT)
(72) Inventor: Bertelli, Vittorio, 20048 Carate Brianaza (IT)
(74) Representative: Gervasi, Gemma

(56) References cited:
- EP-A- 0 345 362
- EP-A- 1 072 252
- WO-A-03/053907
- EMMI S S ET AL: "The Selective OH Radical Oxidation of Sorbitylfurfural: A Combined Experimental and Theoretical Study" JOURNAL OF PHYSICAL CHEMISTRY A, vol. 106, no. 18, 2002, pages 4598-4607, XP002379629 US WASHINGTON, DC

## Description

### Field of the invention

The present invention relates to fatty acid monoesters of sorbityl furfural, as well as relative compositions for cosmetic and dermatological use.

### State of the art

A common cause of skin blemishes, accelerated cellular ageing and many skin diseases is considered to be caused by lowered defences or by an excessive response to free radicals and to the subsequent oxidative phenomena.

An example is the hydroxyl radical OH^{.} that can be generated within the cell during the course of various metabolic processes, including the classical Haber Weiss reaction which consists of the oxidation-reduction between the superoxide anion and hydrogen peroxide in accordance with the following scheme

O₂ +H₂O₂→ OH⁻ + OH +O₂

or the arachidonic cascade in which the hydroxyl radical could be formed when prostaglandin PGG2 is transformed into PGH2 in the presence of the PG-hydroperoxidase enzyme. (M.V. Torrielli, M.U.Dianzani Free Radicals in Inflammatory Disease in Free radicals in Molecular Biology, Aging and Disease D. Armstrong et al. Ed. Raven press, N.Y., 1984).

The hydroxyl radical is characterised by an average life of the order of 0.1 microseconds and by a high reactivity with molecules of fundamental importance present in various biochemical centres such as proteins, nucleic acids or cell membranes. In this latter case the reaction with membrane polyunsaturated fatty acids can be one of the principal causes of the lipoperoxidation responsible for cellular damage and the origin of phlogistic processes.

The possibility of a compound interacting with said type of radical surmises its use as an agent protective against cellular ageing, an event which is closely linked to the presence of free radicals of which the hydroxyl radical certainly represents one of the more active species.

It has been demonstrated that sorbityl furfural characterised by the following formula (II) 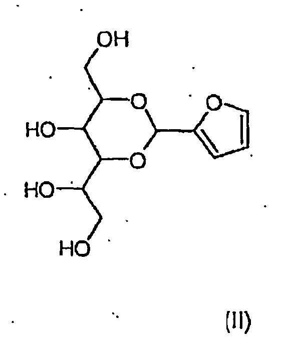 whose use in formulations for cosmetic and dermatological use is described in EP 345362, displays selective antiradical activity towards the hydroxyl radical, as reported by Emmi,S.S.; D'Angelantonio, M.; Poggi, G.; Russo M., Beggiato, G.; Larsen, B.; J. Phys. Chem, A105 (2002) 4598-4607.

By virtue of its specific property of selectively inhibiting the hydroxyl radical, sorbityl furfural can be the active component of a preparation characterised by lenitive action against skin irritations caused by phlogosis.

Indeed, sorbityl furfural has been utilized in a cosmetic cream such as Lichtena® in combination with other active principles such as glycyrrhetic acid and its phytosome, enabling the gradual release of glycyrrhetic acid having inhibitory action on capillary impermeability and on inflammatory infiltrates, thus exerting a bacteriostatic action, α-bisabolol to which some of chamomile's calming properties are attributed and at the concentrations used in said formulations also exerting bacteriostatic activity, and finally allantoin, a stimulant of healthy tissue development able to rapidly digest necrotic tissue and having a keratoplastic and hence natural peeling effect.

The lenitive and protective action of Lichtena® against unfavourable environmental conditions is considerable particularly in cases of erythematous peeling skin [G. Coglio; E. Borgonovo How & Why in Medicine Year I, Supplement I, March 1992 pages 3-7].

A presentation given at the XVIII Congress of the International Federation of Societies of Cosmetic Chemists (Venice, 3-6/10/1994: Gers-Barlag, H.; Mueller, A.;Uhlmann, B.; Sauermann, G.; Wittern, K. P. "Fural Glucitol and Vitamin E Acetate. An appropriate, antioxidative combination in cosmetic products") indicated that combinations of the hydrosoluble antiradical sorbityl furfural and the liposoluble antioxidant vitamin E acetate increased the photoprotective effect of the single components from 0.5 to 2 hours. A combination of the aforesaid active principles for preparing cosmetic and dermatological formulations to protect skin from oxidative stresses was therefore suggested.

Based on the preceding observations regarding the lenitive and anti-reddening action of preparations in which sorbityl furfural is present, the specific anti hydroxyl radical activity of this hydrophilic agent could be enhanced if, in the same preparation, a lipophilic compound were introduced also characterised by specific anti hydroxyl radical activity. The presence of two anti hydroxyl radical agents in the new preparation, one hydrophilic and one lipophilic, would enable this important antiradical action, previously limited to the aqueous phase, to be extended to the non-aqueous (lipophilic) phase.

### Description of the invention

The present invention relates to fatty acid monoesters of sorbityl furfural selected from those of formula (I) and (I') 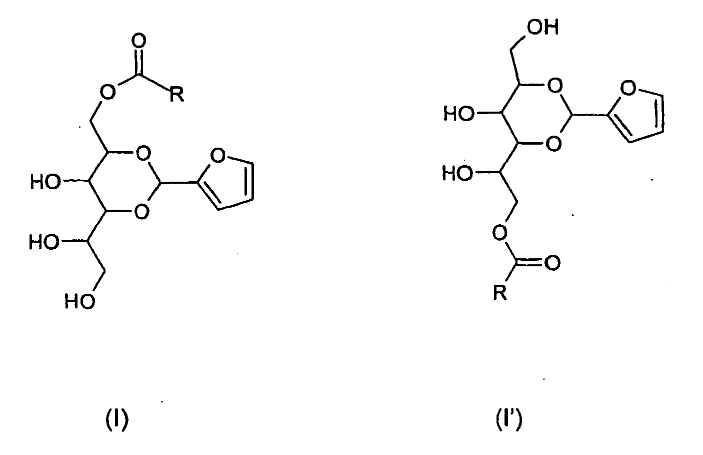 in which R is a linear or branched C₃-C₁₉ alkyl radical optionally containing at least one ethylenic unsaturation.

Preferred among these monoesters in accordance with the present invention are the sorbityl furfural monooctanoates characterised by respectively the following formulas (IA) and (I'A) and mixtures thereof:

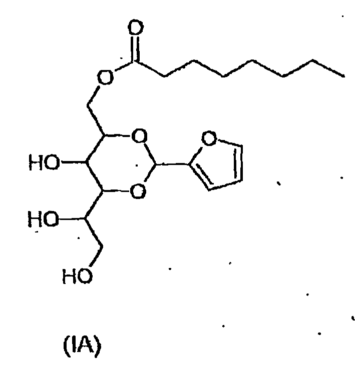

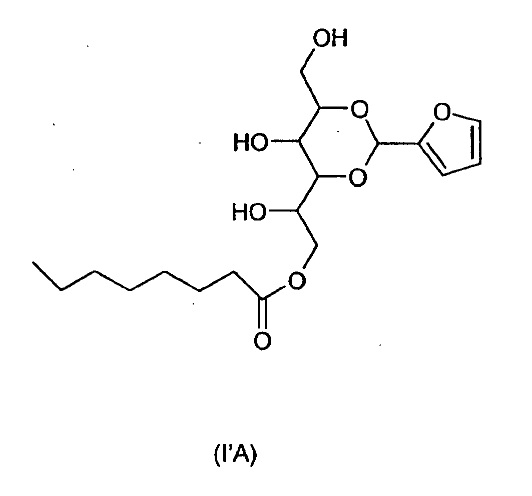

Indeed, the Applicant has found that the liposoluble monoesters of formula (I), (I') and mixtures thereof, in particular sorbityl furfural monooctanoates (IA), (I'A) and mixtures thereof as demonstrated in the *in vitro* trials presented hereinafter, can be advantageously employed as active principles and even more preferably in association with the hydrophilic sorbityl furfural for preparing compositions with lenitive and anti-reddening activity. These latter compositions have the undoubted advantage that the monoesters of the present invention, for example lipophilic sorbityl furfural monooctanoates (IA) and (I'A) and more preferably mixtures thereof exhibit anti hydroxyl radical activity comparable to that of the hydrophilic sorbityl furfural component, and thus exhibit enhanced lenitive and anti-reddening activity when associated with sorbityl furfural compared to formulations described in the state of the art. The compositions of the present invention are preferably in the form of topical formulations suitable for cosmetic and dermatological applications, such as creams, ointments, gels, oils etc., and are suitable for all skin types, especially for sensitive, delicate and easily irritable or irritated skins.

Preferably the monoesters of formula (I) (I') and mixtures thereof can be prepared for example by a process that comprises reacting the sorbityl furfural of formula (II) with an acyl halide of formula (III)

RCOX (III)

or with an acyl anhydride of formula (IIIA):

R(CO)₂O (IIIA)

in which X is a halogen atom chosen from Br and Cl, and R has the aforesaid meanings, in the presence of a hydrogen ion acceptor, preferably consisting of an tertiary aliphatic, alicyclic or aromatic, heterocyclic or heteroaromatic amine, said hydrogen ion acceptor even more preferably consisting of pyridine in a dipolar aprotic solvent preferably consisting of dimethylformamide.

Descriptions are given of an example of the sorbityl furfural monooctanoate preparation in accordance with the present invention and some examples of formulations of the present invention by way of non-limiting illustration, together with *in vitro* trials which show the anti hydroxyl radical activity of the monoesters, in particular sorbityl furfural monooctanoate, in accordance with the present invention.

### EXAMPLE 1 - Preparation of mixtures of sorbityl furfural monooctanoates of formula (IA) and (I'A).

13.0 g (0.05 mol) of sorbityl furfural are suspended in 75 ml of dimethylformamide and 4.1 ml of pyridine. The suspension thus obtained is heated to 40°C until dissolution is complete. 43 ml of octanoyl chloride in 25 ml dimethylformamide are then added dropwise to the solution obtained, previously cooled, over the course of 1 hour.

The mixture is left to react at ambient temperature under mechanical agitation and reaction progress is monitored via TLC (CH₂Cl₂/MeOH, 98:2, v/v).

After 1 hour the spot pertaining to sorbityl furfural has disappeared and the reaction mixture is poured into a solution of 5.0 g sodium bicarbonate in 250 ml of water under mechanical agitation.

After cooling to 0°C for 1 hour a crystalline solid forms which is recovered by filtering under vacuum and dried over calcium chloride. 6.9 g of a yellow solid are obtained, which is crystallized from 30 ml of di-isopropyl ether and 30 ml of toluene. The precipitate is diluted with 30 ml of isopropyl ether at 0°C then filtered under vacuum to obtained 4.9 g of sorbityl furfural monooctanoyl esters (reaction yield: 25.4%) melting point: 118.3°C, UV λ=219 nm. The following ¹H and ¹³CNMR analyses were carried out: COSY (Correlation SpectroscopY), HMQC (Heteronuclear Multiple Quantum Coherence) e TOCSY-HMBC (Total Correlation SpectroscopY-Heteronuclear Multiple Bond Coherence).

A 500 MHz NMR was utilised for carrying out the aforementioned analyses using CDCl₃ as the solvent for ¹H NMR and (CD₃)₂SO for ¹³C NMR.

Said analyses allowed to establish that analysed compound was a mixture consisting of 58%of the compound of formula (I'A) and 42%of the compound (IA). The following signals were attributable to the compound of formula (I'A):
¹H-NMR: δ 7.63 (1 H, m, H-4'), 6.46-4,44 (2H, m, H-2', H-3'), 5,67 (1 H, s, H-A), 4.76-4.74 (2H, m, 2 x OH), 4.45 (1 H, t, J=5.7 Hz, 1-OH), 4.19-4.17 (2H, m, H₂-6), 4.08 (1 H, t, J=5.7 Hz, H-5), 3.70-3.67 (3H, m, H-2, H-3, H-4), 3.56-3.54 (1 H, m, H-1), 3.41-3.39 (1 H, m, H-1), 2.30 (2H, q, J=7.3Hz, COC*H*₂), 1.54-1.51 (2H, m, COCH₂C*H₂*), 1.25-1.23 (8H, m, 4 x C*H₂*), 0.85 (3H, t, J= 6.8 Hz, CH₃).
¹³C-NMR: δ 173.37 (CO), 151.22 (C-1'), 143.02 (C-4'), 110.66 (C-3'), 108.21 (C-2'), 95.01 (C-A), 79.40 (C-4), 78.04 (C-5), 69.45 (C-3), 64.58 (C-6), 62.93 (C-1), 62.20 (C-2), 33.86 (CO*C*H₂), 31.59 (*C*H₂), 28.85 (3 x *C*H₂), 24.93 (*C*H₂), 22.52 (*C*H₂), 14.38 (*C*H₃);
and the following signals to the compound of formula (IA)
¹H-NMR: δ 7.62 (1 H, m, H-4'), 6.46-4,44 (2H, m, H-2', H-3'), 5,64 (1 H, s, H-A), 5.05 (1 H, d, J=5.9 Hz, 3-OH), 4.70 (1 H, t, J=5.6 Hz, 6-OH), 4.48 (1 H, d, J=7.4Hz, 5-OH), 4.22-4.20 (1 H, m, H-1), 3.96-3.95 (2H, m, H-1, H-3), 3.80 (1 H, t, J=6.6 Hz, H-5), 3.70-3.67 (2H, m, H-2, H-4), 3.60-3.57 (2H, m, H₂-6), 2.30 (2H, q, J=7.3Hz, COC*H₂*), 1.54-1.51 (2H, m, COCH₂C*H₂*), 1.25-1.23 (8H, m, 4 x C*H₂*), 0.85 (3H, t, J= 6.8 Hz, CH₃).
¹³C-NMR: δ 173.46 (CO), 151.33 (C-1'), 142.96 (C-4'), 110.66 (C-3'), 108.11 (C-2'), 95.21 (C-A), 81.24 (C-5), 80.20 (C-4), 66.31 (C-3), 66.22 (C-6), 61.62 (C-1), 61.25 (C-2), 34.01 (CO*C*H₂), 31.59 (*C*H₂), 28.85 (3 x *C*H₂), 24.93 (*C*H₂), 22.52 (*C*H₂), 14.38 (*C*H₃).

Elemental analysis of the product of empirical formula C₁₉H₃₀O₈ (theoretical) C 59.06%, H 7.77% (experimental C: 59.12%, H 7.86%).

The mass spectrum obtained by direct injection of the sample (Hewlett Packard 5988A spectrometer, electron impact with electron voltage 70 eV) provided a principal fragment corresponding to the molecular weight of the sorbityl furfural monooctanoate product (M⁺ at m/z 386) and other fragmentations, the most significant of which being at 229 m/z (M⁺ - C₉H₁₇O₂) corresponding to the detachment of a fragment CH₃-(CH₂)₆-COOCH₂⁺ and at m/z 127 corresponding to the fragment CH₃-(CH₂)₆-CO⁺; the peak at m/z 97 probably corresponds to the fragment containing the furan ring C₅H₅O₂⁺.

### Solubility:

50 mg insolubles in 5 ml (<1 %) of water;
50 mg in n-octanol: soluble in 6 ml while hot*
50 mg in triolein: soluble in 8 ml while hot*
50 mg in 1,2-propanediol: soluble in 1 ml while hot (5%)*
50 mg in glycerol: soluble in 6 ml while hot (1.6%)*,
* once dissolved under hot conditions it remains in solution even at ambient temperature.

### ANTIRADICAL ACTIVITY OF SORBITYL FURFURAL MONOOCTANOATE (IA)/(I'A) COMPARED WITH SORBITYL FURFURAL (II)

### Deoxyribose method (Aruoma)

The model enables calculation of the reaction constant of the molecule towards the OH radical, following the method described by Aruoma (I.O. Aruoma , Meth. Enzymol., 1994, vol. 233, 57-66). The reaction between Fe³⁺ EDTA and H₂O₂ in the presence of ascorbic acid generates a hydroxyl radical which attacks the deoxyribose to form a product which, with thiobarbituric acid under hot conditions, develops a pink chromophore with maximum absorbance at 532 nm. In the presence of a substance able to react with the hydroxyl radical there is a reduction in chromophore formation. By way of a simple equation whereby the reaction constant of deoxyribose towards the hydroxyl radical is known, the reaction constant of the molecule under examination can be calculated. The greater this value, the greater will be its ability to capture the hydroxyl radical. The obtained results, given in the following Table (1), were obtained within the concentration range 0.01 to 1 mM for the mixture of compounds (IA) and (I'A), and (II) and indicate that sorbityl furfural and the corresponding mixture of fatty acid monoesters (IA) and (I'A) exhibit comparable anti hydroxyl radical activity.

**TABLE 1**

| | |
|---|---|
| sorbityl furfural (II) | K_{S}= 7.14±0.62 x 10⁹ |
| Mixture of sorbityl furfural monooctanoates (IA) and (I'A) | K_{S}=6.62±0.57 x 10⁹ |

| Formulation 1 for cream | |
|---|---|
| Demineralized water | remainder to 100 |
| Edetate disodium | 0.150% |
| Carbomer | 0.450% |
| Panthenol | 1.000% |
| Sodium dehydroacetate | 0.200% |
| Sorbityl furfural | 0.700% |
| Triethanolamine | quantity required to achieve pH 6.0-7.0 |
| Beeswax PEG-8 | 12.000% |
| Caprylic/capric triglyceride | 8.000% |
| Isostearyl isostearate | 6.000% |
| Shea butter | 2.000% |
| Paraben mixture | 0.300% |
| Squalane | 3.000% |
| Tocopherol | 0.200% |
| Ascorbyl palmitate | 0.003% |
| Mixture of (IA) and (I'A) | |
| (Sorbityl furfural monooctanoates) | 0.700% |
| Glycyrrhetic acid | 0.200% |
| Bisabolol | 0.300% |

| Formulation 2 | |
|---|---|
| Demineralized water | remainder to 100 |
| Edetate disodium | 0.150 % |
| Carbomer | 0.450 % |
| Panthenol | 1.000 % |
| Triethanolamine | quantity required to achieve pH 6.0-7.0 |
| Beeswax PEG-8 | 12.000 % |
| Caprylic/capric triglyceride | 8.000 % |
| Isostearyl isostearate | 6.000 % |
| Shea butter | 2.000 % |
| Vegetable oil | 2.000 % |
| Squalane | 3.000% |
| Tocopherol | 0.200 % |
| Ascorbyl palmitate | 0.003% |
| Mixture of (IA) and (I'A) | |
| (Sorbityl furfural monooctanoates) | 0.700% |
| Glycyrrhetic acid | 0.200% |
| Bisabolol | 0.300% |
| Preservative system | in the required quantity |

## Claims

1. Fatty acid monoesters of sorbityl furfural, selected from those of formula (I) and (I') 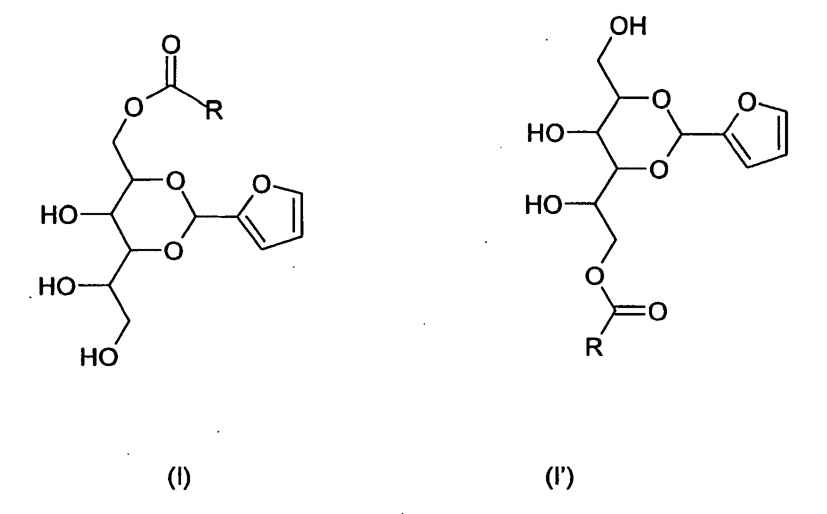 in which R is a linear or branched C₃-C₁₉ alkyl radical optionally containing at least one ethylenic unsaturation.

2. The monoester as claimed in claim 1, **characterised by** being the sorbityl furfural monooctanoates selected from those of formula (IA) and (I'A)

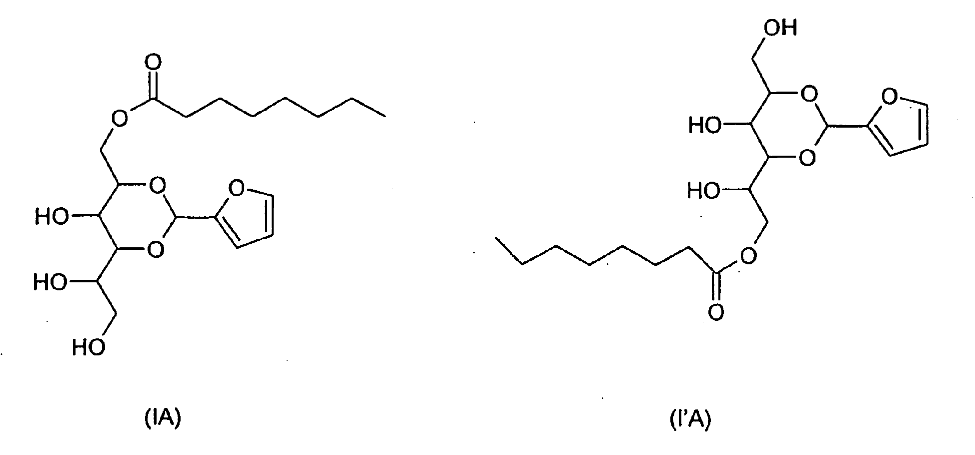

3. A composition having lenitive and anti-reddening activity comprising as active principle at least one fatty acid monoester of sorbityl furfural as claimed in claim 1 or 2 in combination with suitable excipients and/or diluents.

4. The composition according to claim 3 containing a mixture of the compounds according to claim 1.

5. The composition according to anyone of claims 3 and 4, containing a mixture of the compounds according to claim 2.

6. The composition according to anyone of claims 3-5 comprising as lipophilic agent at least one fatty acid monoester as claimed in claim 1 or 2 and sorbityl furfural as hydrophilic agent in combination with suitable excipients and/or diluents.

7. The composition according to anyone of claims 3-6, in the form of topical formulations suitable for dermatological application for all skin types.

8. The composition as claimed in claim 6, **characterised** as suitable for the dermatological treatment of sensitive, delicate, easily irritable or irritated skins.

9. The composition according to anyone of claims 3-8, in the form of creams, ointments, gels or oils.

10. Process for preparing fatty acid monoesters of sorbityl furfural as claimed in claim 1 comprising the reaction of the sorbityl furfural of formula (II) 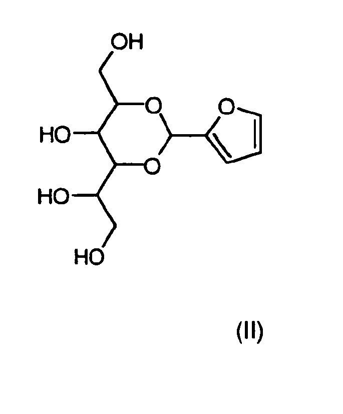 with an acyl halide of formula (III)
RCOX (III)
or with an acyl anhydride of formula (IIIA):
R(CO)₂O (IIIA)
in which X is a halogen atom chosen from Br and Cl, and R has the aforesaid meanings, in the presence of a hydrogen ion acceptor in a dipolar aprotic solvent.

11. The process as claimed in claim 10, **characterised in that** said hydrogen ion acceptor is a tertiary aliphatic, alicyclic, aromatic, heterocyclic or heteroaromatic amine.

12. The process as claimed in claim 11, **characterised in that** said tertiary amine is pyridine.

13. The process as claimed in any one of claims 10-12, **characterised in that** said dipolar aprotic solvent is dimethylformamide.

14. The process according to anyone of claim 10-13 wherein when R=n-heptyl a mixture of monoesters of formula (IA) and (I'A) in molar or weight ratio 1:1 is obtained.

## Patentansprüche

1. Fettsäuremonoester von Sorbitylfurfural ausgewählt aus solchen der Formel (I) und (I') 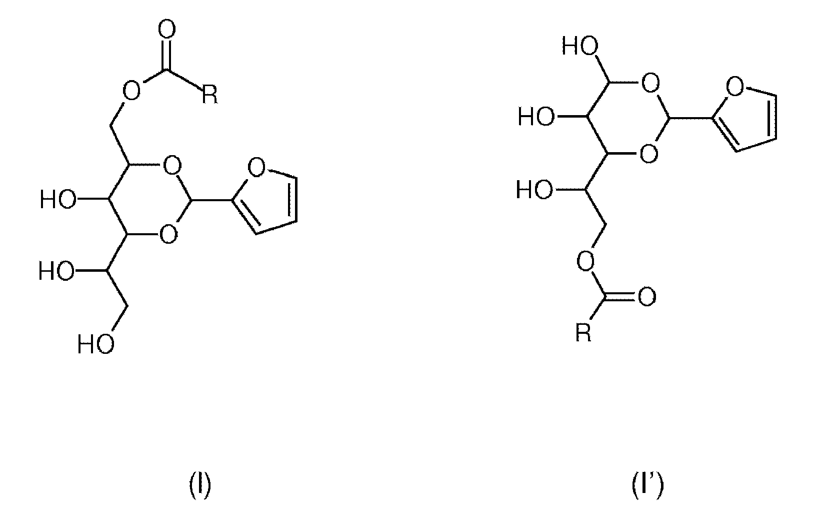 worin R ein lineares oder verzweigtes C₃-C₁₉-Alkylradikal ist, welches optional wenigstens eine ethylenische Ungesättigtheit enthält.

2. Monoester nach Anspruch 1, **dadurch gekennzeichnet, dass** dieser das Sorbitylfurfuralmonooctanoat ausgewählt aus solchen der Formeln (IA) und (I'A) ist:

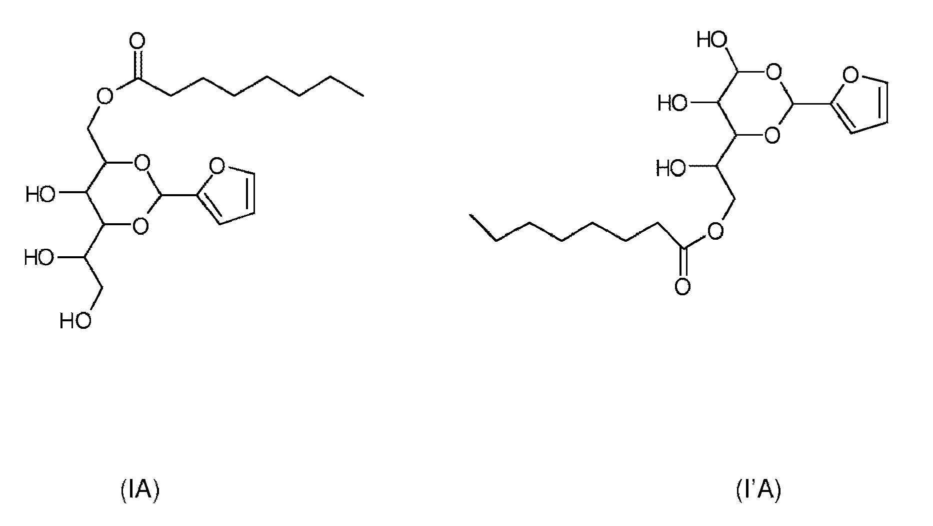

3. Zusammensetzung mit einer lindernden und einer antirötenden Aktivität, welche als aktiven Hauptbestandteil wenigstens einen Fettsäuremonoester von Sorbitylfurfural nach Anspruch 1 oder 2 in Mischung mit geeigneten Hilfsstoffen und/oder Verdünnungsmitteln enthält.

4. Zusammensetzung nach Anspruch 3, welche eine Mischung der Verbindungen nach Anspruch 1 enthält.

5. Zusammensetzung nach Anspruch 3 oder 4, welche eine Mischung der Verbindungen nach Patentanspruch 2 enthält.

6. Zusammensetzung nach einem der Ansprüche 3 bis 5, welche als lipophiles Mittel wenigstens einen Fettsäuremonoester nach Anspruch 1 oder 2 und Sorbitylfurfural als hydrophiles Mittel in Mischung mit geeigneten Hilfsmitteln und/oder Verdünnungsmitteln enthält.

7. Zusammensetzung nach einem der Ansprüche 3 bis 6 in der Form von topischen Formulierungen geeignet für die dermatologische Anwendung für alle Hauttypen.

8. Zusammensetzung nach Anspruch 6, **dadurch gekennzeichnet, dass** diese für die dermatologische Behandlung von sensitiver, feinfühliger, leicht reizbarer oder gereizter Haut geeignet ist.

9. Zusammensetzung nach einem der Ansprüche 3 bis 8 in der Form von Creme, Salbe, Gel oder Öl.

10. Verfahren zum Herstellen von Fettsäuremonoestern von Sorbitylfurfural nach Anspruch 1 umfassend die Reaktion eines Sorbitylfurfurals gemäß der Formel (II) 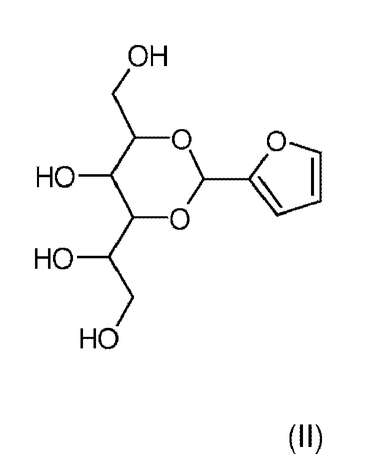 mit einem Acylhalogenid der Formel (III)
RCOX (III)
oder mit einem Acylanhydrid der Formel (IIIA):
R(CO)₂O (IIIA)
worin X ein Halogenatom ausgewählt aus Br und Cl ist und R die vorgenannten Bedeutungen aufweist, in der Gegenwart eines Wasserstoffionenakzeptors in einem dipolaren aprotischen Lösungsmittel.

11. Verfahren nach Anspruch 10, **dadurch gekennzeichnet, dass** der Wasserstoffionenakzeptor ein tertiäres, aliphatisches, alicyclisches, aromatisches, heterozyklisches oder heteroaromatisches Amin ist.

12. Verfahren nach Anspruch 11, **dadurch gekennzeichnet, dass** das tertiäre Amin Pyridin ist.

13. Verfahren nach einem der Ansprüche 10 bis 12, **dadurch gekennzeichnet, dass** das dipolare aprotische Lösungsmittel Dimethylformamid ist.

14. Verfahren nach einem der Ansprüche 10 bis 13, wobei, wenn R = n-Heptyl ist, eine Mischung aus Monoestern der Formeln (IA) und (I'A) in einem molaren Verhältnis oder Gewichtsverhältnis von 1:1 erhalten wird.

## Revendications

1. Monoesters d'acide gras de sorbityl furfural, choisis parmi ceux de formule (I) et (I') 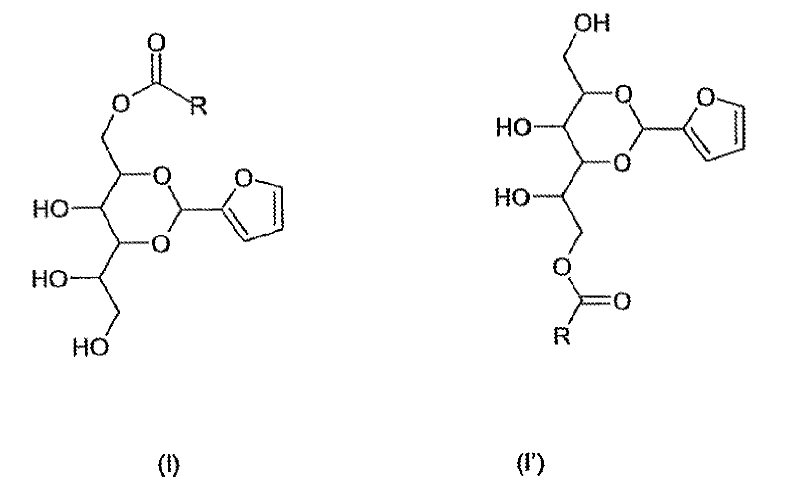 où R est un radical alkyle en C₃ à C₁₉ linéaire ou ramifié contenant facultativement au moins une insaturation éthylénique.

2. Monoester selon la revendication 1, **caractérisé en ce qu'**il s'agit des monooctanoates de sorbityl furfural choisis parmi ceux de formule (IA) et (I'A)

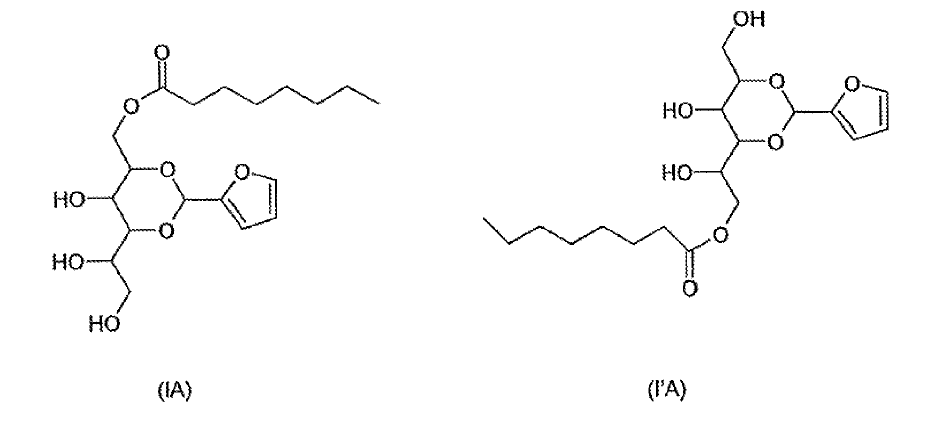

3. Composition ayant une activité lénitive et anti-rougeur comprenant comme principe actif au moins un monoester d'acide gras de sorbityl furfural selon la revendication 1 ou 2 en combinaison avec des excipients et/ou des diluants appropriés.

4. Composition selon la revendication 3, contenant un mélange des composés selon la revendication 1.

5. Composition selon l'une quelconque des revendications 3 et 4, contenant un mélange des composés selon la revendication 2.

6. Composition selon l'une quelconque des revendications 3 à 5, comprenant comme agent lipophile au moins un monoester d'acide gras selon la revendication 1 ou 2 et du sorbityl furfural comme agent hydrophile en combinaison avec des excipients et/ou des diluants appropriés.

7. Composition selon l'une quelconque des revendications 3 à 6, sous la forme de formulations topiques appropriées pour une application dermatologique destinée à tous les types de peau.

8. Composition selon la revendication 6, **caractérisée** comme appropriée pour le traitement dermatologique des peaux sensibles, délicates, facilement irritables ou irritées.

9. Composition selon l'une quelconque des revendications 3 à 8, sous la forme de crèmes, de pommades, de gels ou d'huiles.

10. Procédé de préparation de monoesters d'acide gras de sorbitol furfural selon la revendication 1, comprenant la réaction du sorbityl furfural de formule (II) 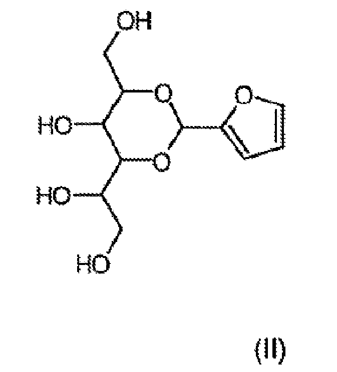 avec un halogénure d'acyle de formule (III)
RCOX (III)
ou avec un anhydride d'acyle de formule (IIIA) :
R(CO)₂O (IIIA)
où X est un atome d'halogène choisi parmi Br et Cl et R a les significations précitées, en présence d'un accepteur d'ion hydrogène dans un solvant aprotique dipolaire.

11. Procédé selon la revendication 10, **caractérisé en ce que** ledit accepteur d'ion hydrogène est une amine tertiaire aliphatique, alicyclique, aromatique, hétérocyclique ou hétéroaromatique.

12. Procédé selon la revendication 11, **caractérisé en ce que** ladite amine tertiaire est la pyridine.

13. Procédé selon l'une quelconque des revendications 10 à 12, **caractérisé en ce que** ledit solvant aprotique dipolaire est le diméthylformamide.

14. Procédé selon l'une quelconque des revendications 10 à 13, dans lequel, lorsque R = n-heptyle, un mélange de monoesters de formule (IA) et (I'A) en un rapport molaire ou en poids de 1 : 1 est obtenu.
